# EUROPEAN PATENT APPLICATION

(11) **EP 2 617 683 A1**
(43) Date of publication of application: **24.07.2013**
(21) Application number: 11825085.1
(22) Date of filing: 09.09.2011
(51) Int. Cl.: C01G 9/02, A61K 8/27, A61Q 17/04

(54) **ZINC OXIDE PARTICLES AND COSMETIC MATERIAL**

(30) Priority: 13.09.2010 JP 2010204745
(71) Applicant: Sakai Chemical Industry Co., Ltd., Sakai-shi, Osaka 590-8502 (JP)
(72) Inventor: HASHIMOTO, Mitsuo, Iwaki-shi Fukushima 971-8183 (JP); HAKOZAKI, Hiroshi, Iwaki-shi Fukushima 971-8183 (JP)
(74) Representative: Isarpatent
(86) International application number: PCT/JP2011/070588
(87) International publication number: WO 2012/036082

(57) **Abstract**

An object of the present invention is to provide large-sized zinc oxide particles that show, when incorporated to a cosmetic product, excellent properties including transparency, infrared reflection, and the like without deteriorating the feel; and also a cosmetic containing the same.

The zinc oxide particles having an average particle diameter of 3 to 20 µm, an average friction coefficient of 3 or less, a total visible light transmittance of 85% or more, and a near-infrared reflectance of 80% or more.

## Description

### TECHNICAL FIELD

The present invention relates to zinc oxide particles and a cosmetic.

### BACKGROUND OF THE DISCLOSURE

In the field of cosmetic applications, zinc oxide particles have been used mainly as a UV absorber for sun screen products. In most cases, zinc oxide particles for such applications are ultrafine particles of 100 nm or less. However, zinc oxide particles are rarely used for skin-care cosmetics such as skin lotions and milky lotions or makeup cosmetics such as foundations, lip sticks, and eye shadows.

In such skin-care cosmetics and makeup cosmetics, the feel is regarded as important. It is also important that they do not give a whitish finish when applied to the skin. Therefore, particles that are excellent in terms of these functions, such as talc, sericite, platy barium sulfate, and boron nitride, have been used in skin-care cosmetics or makeup cosmetics.

It is known to incorporate zinc oxide in order to impart UV absorption ability to a skin-care cosmetic or a makeup cosmetic (Patent Documents 1, 2, and 3). However, in the case where zinc oxide particles are ultrafine particles of 100 nm or less, there has been a problem in that they cause squeakiness, resulting in the deterioration of the feel. Furthermore, in the case where zinc oxide particles are 100 nm to 3 µm, in addition to the same problem as in the case of using ultrafine zinc oxide particles, there has also been a problem in that transparency is significantly impaired, resulting in a whitish finish. In addition, in terms of effects on the skin, not only ultraviolet light but also near-infrared light has been increasingly regarded as a problem. Therefore, cosmetics capable of blocking near-infrared light have been demanded.

Patent Document 4 discloses a cosmetic containing zinc oxide of 0.5 to 20 µm as an IR-blocking substance. However, such zinc oxide particles have a large average friction coefficient. Therefore, there has been a problem in that when incorporated in a large amount, they cause squeakiness, resulting in a product that is uncomfortable to use. In addition, Patent Document 4 does not disclose transparency. In addition, the zinc oxide particles and titanium oxide particles have a problem in that their total visible light transmittance is low, which leads to low transparency and a whitish finish.

In addition, zinc oxide particles having a particle diameter of more than 3 µm are known (Patent Documents 5 and 6). However, there is no description about the incorporation of such zinc oxide particles to a cosmetic. Further, the performance resulting from incorporation to a cosmetic is not disclosed.

### PRIOR TECHNICAL DOCUMENT

### PATENT DOCUMENT

[Patent Document 1] Japanese Kokai Publication Hei02-289506
[Patent Document 2] Japanese Kokai Publication Sho61-236708
[Patent Document 3] Japanese Kokai Publication Hei07-118133
[Patent Document 4] Japanese Kokai Publication 2005-162695
[Patent Document 5] Japanese Kokai Publication 2008-218749
[Patent Document 6] Japanese Kokai Publication 2009-249226

### SUMMARY OF INVENTION

### PROBLEMS TO BE SOLVED BY THE INVENTION

In view of the above-mentioned state, an object of the present invention is to provide large-sized zinc oxide particles that show, when incorporated to a cosmetic product, excellent properties including transparency, infrared reflection, and the like without deteriorating the feel; and also a cosmetic containing the same.

### MEANS FOR SOLVING THE PROBLEMS

The present invention provides zinc oxide having an average particle diameter of 3 to 20 µm, an average friction coefficient of 3 or less, a total visible light transmittance of 85% or more, and a near-infrared reflectance of 80% or more.
The present invention also provides a cosmetic comprising the zinc oxide particles mentioned above.

### EFFECTS OF THE INVENTION

The zinc oxide particles of the present invention have an average particle diameter of 3 to 20 µm, an average friction coefficient of 3 or less, a total visible light transmittance of 85% or more, and a near-infrared reflectance of 80% or more. A cosmetic containing the zinc oxide particles is comfortable to use and has excellent transparency. At the same time, owing to the effects of near-infrared reflection property and the heat releasing property of zinc oxide, the cosmetic has excellent cooling sensation and lasts long.

### MODE FOR CARRYING OUT THE INVENTION

The present invention will be described in detail hereinafter.
The zinc oxide particles of the present invention are characterized by having an average particle diameter of 3 to 20 µm, an average friction coefficient of 3 or less, a total visible light transmittance of 85% or more, and a near-infrared reflectance of 80% or more. Most of zinc oxide particles conventionally incorporated to cosmetics are less than 3 µm, and it has been impossible to satisfy all of the various functions mentioned above. The various characteristics in the present invention have been found by the present inventors.

The shape of the zinc oxide particles of the present invention is not particularly restricted, but a shape that is closer to spherical is preferable. It is more preferable that the zinc oxide particles of the present invention have an average particle diameter within a range of 3 to 15 µm. Average particle diameter herein is a value measured using a laser diffraction/scattering particle size distribution analyzer (manufactured by Beckman Coulter, Inc., Model LS 13 320).

In the zinc oxide particles, an average particle diameter below the above range results in an increased squeaky texture and deteriorates the feel, and thus is undesirable. In addition, an average particle diameter over the above range results in roughness and thus is undesirable.

The zinc oxide particles of the present invention have an average friction coefficient of 3 or less. Average friction coefficient herein is measured by the method described in the Examples. Zinc oxide particles having an average friction coefficient of greater than 3 cause squeakiness when incorporated to a cosmetic, so the obtained product is uncomfortable to use.

The average friction coefficient of zinc oxide particles greatly varies depending on the particle diameter, particle shape, particle surface conditions, etc. In order to reduce the friction coefficient to obtain the zinc oxide particles of the present invention, it is preferable that the particle diameter is large, the particle shape is smooth, and the surface has little irregularities. The zinc oxide particles of the present invention have such low-friction property. Therefore, when used in a cosmetic, excellent using comfort can be obtained.

The zinc oxide particles of the present invention have a total visible light transmittance of 85% or more. A total visible light transmittance of less than 85% gives a whitish finish and thus is undesirable. Incidentally, the total visible light transmittance is a value measured by the measurement method described in the Examples below. In the present invention, "a total visible light transmittance of 85% or more" means that in the case where the measurement is performed at two wavelengths, 400 nm and 550 nm, the results are each 85% or more.

The zinc oxide particles of the present invention have a near-infrared reflectance of 80% or more. Near-infrared light has thermal energy. Therefore, when infrared light can be reflected on the skin by a cosmetic, there is an advantage in that the heat can be reduced in summer, thereby maintaining skin comfortability. In addition, it has been suggested that near-infrared light may cause skin problems. Accordingly, the blocking of near-infrared light is desirable also from skin physiological point of view.

Incidentally, the near-infrared reflectance is a value measured by the measurement method described in the Examples below. In the present invention, "a near-infrared reflectance of 80% or more" means that reflectance measured at 1000 nm and reflectance measured at 2000 nm are each 80% or more.

The zinc oxide particles of the present invention are not particularly restricted in terms of production method and may be produced, for example, by calcinating zinc oxide at 800 to 1200°C. The calcination apparatus and the calcination time can be suitably selected according to the desired size of zinc oxide particles, etc.

In the production method mentioned above, the calcination is performed at 800 to 1200°C. As a result, zinc oxide particles are fused, forming zinc oxide particles having a large particle diameter. In addition, presumably, surface irregularities are eliminated by calcination, forming zinc oxide particles having a small average friction coefficient.

In the production method mentioned above, raw-material zinc oxide particles, which are used as a raw material, are not particularly restricted, and those having an average particle diameter of 0.01 to 1.0 µm may be used. Examples of commercially available zinc oxide particles include FINEX-75, FINEX-50, FINEX-30, fine zinc oxide, SF-15, Zinc Oxide No. 1, and the like manufactured by Sakai Chemical Industry Co., Ltd.

When the reaction temperature is less than 800°C, it may be impossible to obtain zinc oxide particles having a sufficiently large particle diameter of 3 µm or more. When the reaction temperature is 1200°C or more, this may lead to the increased formation of coarse particles, resulting in a decrease in yield. Therefore, this is undesirable.

The zinc oxide particles produced by the above method may be classified by crushing and sieving. The crushing method is not particularly restricted, and an atomizer or the like may be used, for example. Examples of methods for classification by sieving include wet classification and dry classification.

The zinc oxide particles of the present invention may be surfacetreated as necessary. The surface treatment may be performed by an ordinary treatment method used in the technical field of inorganic particles. More specifically, examples thereof include organic surface treatments with a silane coupling agent, silicone oil, etc., and inorganic surface treatment with silica, etc.

In addition, the present invention also relates to a cosmetic containing the zinc oxide particles. It is particularly preferable that the cosmetic of the present invention is a makeup cosmetic. Specific examples thereof include base-makeup cosmetics such as foundations and face powders and point-makeup cosmetics such as eye shadows and blushes. Examples also include skin-care cosmetics such as creams, milky lotions, and skin lotions.

The cosmetic of the present invention is not particularly restricted in terms of the amount of the zinc oxide particles incorporated, but it is preferable that the cosmetic contains the zinc oxide particles in an amount of 0.5 to 50 wt%.

The cosmetic of the present invention may be used together with any color pigments, extenders, aqueous components, and oily components usable in the field of cosmetics.

Examples of color pigments include, but are not particularly restricted to, inorganic white pigments (e.g., titanium dioxide, etc.); inorganic red pigments (e.g., iron oxide (red oxide), iron titanate, etc.); inorganic brown pigments (e.g., γ-iron oxide, etc.); inorganic yellow pigments (e.g., yellow iron oxide, yellow ocher, etc.); inorganic black pigments (e.g., black iron oxide, lower-order titanium oxide, etc.); inorganic purple pigments (e.g., mango violet, cobalt violet, etc.); inorganic green pigments (e.g., chrome oxide, chromium hydroxide, cobalt titanate, etc.); inorganic blue pigments (e.g., ultramarine blue, iron blue, etc.); pearl pigments (e.g., titanium oxide-coated mica, titanium oxide-coated bismuth oxychloride, titanium oxide-coated talc, a colored titanium oxide-coated mica, bismuth oxychloride, argentine, etc.); metal powder pigments (e.g., aluminum powder, cupper powder, etc.); organic pigments such as zirconium, barium, and aluminum lake (e.g., organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404, Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, Blue No. 1, etc.); and natural pigments (e.g., chlorophyll, β-carotene, etc.). Examples of the extenders include, but are not particularly restricted to, mica, synthetic mica, sericite, talc, kaolin, calcium carbonate, magnesium carbonate, barium sulfate, and aluminum oxide.

Examples of the aqueous components and the oily components include, but are not particularly restricted to, those containing components such as oils, surfactants, moisturizers, higher alcohols, sequestrants, natural and synthetic polymers, water-soluble and oil-soluble polymers, UV absorbers, various extracts, inorganic and organic pigments, inorganic and organic clay minerals, inorganic and organic pigments treated with metal soap or silicone, coloring materials such as organic dyes, preservatives, antioxidants, dyes, thickeners, pH adjusters, perfumes, cooling agents, antiperspirants, disinfectants, and skin activators. Specifically, a desired cosmetic can be produced in the usual manner using any one or more of the components listed below. The amounts of these components incorporated are not particularly restricted as long as they do not interfere with the effects of the present inveniton.

Examples of the oils include, but are not particularly restricted to, avocado oil, camellia oil, turtle oil, macadamia nut oil, corn oil, mink oil, olive oil, rapeseed oil, egg-yolk oil, sesame oil, persic oil, wheat germ oil, sasanqua oil, castor oil, linseed oil, safflower oil, cotton seed oil, perilla oil, soybean oil, arachis oil, tea seed oil, kaya oil, rice bran oil, Chinese tung oil, Japanese tung oil, jojoba oil, germ oil, triglycerol, glyceryl trioctanoate, glyceryl triisopalmitate, cacao butter, coconut oil, horse fat, hydrogenated coconut oil, palm oil, beef tallow, mutton tallow, hydrogenated beef tallow, palm kernel oil, lard, beef bone fat, Japan wax kernel oil, hydrogenated oil, neatsfoot oil, Japan wax, hydrogenated castor oil, beeswax, candelilla wax, cotton wax, carnauba wax, bayberry wax, insect wax, spermaceti wax, montan wax, bran wax, lanolin, kapok wax, lanolin acetate, liquid lanolin, sugarcane wax, isopropyl lanolate, hexyllaurate, reduced lanolin, jojoba wax, hard lanolin, shellac wax, POE lanolin alcohol ether, POE lanolin alcohol acetate, POE cholesterol ether, polyethylene glycol lanolate, POE hydrogenated lanolin alcohol ether, liquid paraffin, ozokerite, pristane, paraffin, ceresin, squalene, Vaseline, and microcrystalline wax.

Examples of lipophilic nonionic surfactants include, but are not particularly restricted to, sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; (poly)glycerol esters of fatty acids such as glycerol mono-cotton seed oil fatty acid ester, glyceryl monoerucate, glyceryl sesquioleate, glyceryl monostearate, α,α'-glyceryl oleate pyroglutamate, and glyceryl monostearate malate; propylene glycol fatty acid esters such as propylene glycol monostearate; hydrogenated castor oil derivatives; and glycerol alkyl ethers.

Examples of hydrophilic nonionic surfactants include, but are not particularly restricted to, POE sorbitan fatty acid esters such as POE-sorbitan monooleate, POE-sorbitan monostearate, POE-sorbitan monooleate, and POE-sorbitan tetraoleate; POE sorbitol fatty acid esters such as POE-sorbitol monolaurate, POE-sorbitol monooleate, POE-sorbitol pentaoleate, and POE-sorbitol monostearate; POE glycerol fatty acid esters such as POE-glycerol monostearate, POE-glycerin monoisostearate, and POE-glycerol triisostearate; POE fatty acid esters such as POE monooleate, POE distearate, POE monodioleate, and ethylene glycol distearate; POE alkyl ethers such as POE lauryl ether, POE oleyl ether, POE stearyl ether, POE behenyl ether, POE2-octyldodecyl ether, and POE cholestanol ether; POE alkylphenyl ethers such as POE octylphenyl ether, POE nonylphenyl ether, and POE dinonylphenyl ether; pluaronic types such as Pluronic; POE-POP alkyl ethers such as POE-POP cetyl ether, POE-POP 2-decyl tetradecyl ether, POE-POP monobutyl ether, POE-POP hydrogenated lanolin, and POE-POP glycerol ether; tetra POE-tetra POP ethylenediamine condensates such as Tetronic; POE castor oil or hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamate monoisostearic acid diester, and POE hydrogenated castor oil maleate; POE beeswax-lanolin derivatives such as POE sorbitol beeswax; and alkanolamides such as coconut oil fatty acid diethanolamide, lauric acid monoethanolamide, fatty acid isopropanolamide, POE propylene glycol fatty acid esters, POE alkylamines, POE fatty acid amides, sucrose fatty acid esters, POE nonylphenyl formaldehyde condensates, alkyl ethoxydimethylamine oxides, and trioleyl phosphate.

Examples of other surfactants include anionic surfactants such as fatty acid soaps, higher-alkyl sulfuric ester salts, POE triethanolamine lauryl sulfate, and alkyl ether sulfuric ester salts; cationic surfactants such as alkyl trimethylammonium salts, alkyl pyridinium salts, alkyl quaternary ammonium salts, alkyl dimethylbenzyl ammonium salts, POE alkylamines, alkylamine salts, and polyamine fatty acid derivatives; and amphoteric surfactants such as imidazoline amphoteric surfactants and betaine surfactants. They may be incorporated within a range where they do not cause any problems with stability and skin irritation.

Examples of the moisturizers include, but are not particularly restricted to, xylitol, sorbitol, maltitol, chondroitin sulfate, hyaluronic acid, mucoitinsulfuric acid, caronic acid, atelocollagen, cholesteryl-12-hydroxystearate, sodium lactate, bile salts, dl-pyrrolidone carboxylate, short-chain soluble collagens, diglycerol (EO) PO adducts, Rosa roxburghii extract, yarrow extract, and melilot extract.

Examples of the higher alcohols include, but are not particularly restricted to, linear alcohols such as lauryl alcohol, cetyl alcohol, stearyl alcohol, behenyl alcohol, myristyl alcohol, oleyl alcohol, and cetostearyl alcohol; and branched alcohols such as monostearyl glycerol ether (batyl alcohol), 2-decyltetradecynol, lanolin alcohol, cholesterol, phytosterol, hexyldodecanol, isostearyl alcohol, and octyldodecanol.

Examples of the sequestrants include, but are not particularly restricted to, 1-hydroxyethane-1,1-diphosphonic acid, 1-hydroxyethane-1,1-diphosphonic acid tetrasodium salt, sodium citrate, sodium polyphosphate, sodium metaphosphate, gluconic acid, phosphoric acid, citric acid, ascorbic acid, succinic acid, and edetic acid.

Examples of natural water-soluble polymers include, but are not particularly restricted to, plant-derived polymers such as gum arabic, tragacanth gum, galactan, guar gum, carob gum, karaya gum, carrageenan, pectin, agar, quince seed (quince), algal colloid (algal extract), starch (rice, corn, potato, wheat), and glycyrrhizinic acid; microorganism-derived polymers such as xanthan gum, dextran, succinoglucan, and pullulan; and animal-derived polymers such as collagen, casein, albumin, and gelatin.

Examples of semisynthetic water-soluble polymers include, but are not particularly restricted to, starch polymers such as carboxymethyl starch and methyl hydroxypropyl starch; cellulose polymers such as methyl cellulose, nitro cellulose, ethyl cellulose, methyl hydroxypropyl cellulose, hydroxyethyl cellulose, cellulose sodium sulfate, hydroxypropyl cellulose, sodium carboxymethylcellulose (CMC), crystalline cellulose, and cellulose powder; and alginate polymers such as sodium alginate and propylene glycol alginate.

Examples of synthetic water-soluble polymers include, but are not particularly restricted to, vinyl polymers such as polyvinyl alcohol, polyvinyl methyl ether, and polyvinyl pyrrolidone; polyoxyethylene polymers such as polyethylene glycol 20,000, polyethylene glycol 40,000, and polyethylene glycol 60,000; copolymers such as polyoxyethylene-polyoxypropylene copolymers; acrylic polymers such as sodium polyacrylate, polyethylacrylate, and polyacrylamide; polyethyleneimine; and cationic polymers.

Examples of inorganic water-soluble polymers include, but are not particularly restricted to, bentonite, magnesium aluminum silicate (Veegum), laponite, hectorite, and silicic anhydride.

Examples of UV absorbers include, but are not particularly restricted to, benzoate UV absorbers such as p-aminobenzoic acid (hereafter abbreviated to PABA), PABA monoglycerol ester, N,N-dipropoxy PABA ethyl ester, N,N-diethoxy PABA ethyl ester, N,N-dimethyl PABA ethyl ester, and N,N-dimethyl PABA butyl ester; anthranilate UV absorbers such as homomenthyl-N-acetyl anthranilate; salicylate UV absorbers such as amyl salicylate, menthyl salicylate, homomenthyl salicylate, octyl salicylate, phenyl salicylate, benzyl salicylate, and p-isopropanol phenyl salicylate; cinnamate UV absorbers such as octyl cinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, and glyceryl mono-2-ethylhexanoyl diparamethoxycinnamate; and benzophenone UV absorbers such as 2,4-dihydroxy benzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, 3-(4'-methylbenzylidene)-d,l-camphor, 3-benzylidene-d,l-camphor, urocanic acid, urocanic acid ethyl ester, 2-phenyl-5-methylbenzoxazole, 2,2'-hydroxy-5-methylphenyl benzotriazole, 2-(2'-hydroxy-5'-t-octylphenyl)benzotriazole, 2-(2'-hydroxy-5'-methylphenyl)benzotriazole, dibenzalazine, dianisoyl methane, 4-methoxy-4'-t-butyldibenzoylmethane, and 5-(3,3-dimethyl-2-norbornylidene)-3-pentan-2-one.

Examples of various extracts include, but are not particularly restricted to, Houttuynia cordata extract, Phellodendron bark extract, melilot extract, dead nettle extract, licorice extract, peony root extract, soapwort extract, luffa extract, cinchona extract, strawberry geranium extract, sophora root extract, nuphar extract, fennel extract, primrose extract, rose extract, rehmannia root extract, lemon extract, lithospermum root extract, aloe extract, calamus root extract, eucalyptus extract, field horsetail extract, sage extract, thyme extract, tea extract, seaweed extract, cucumber extract, clove extract, bramble extract, lemon balm extract, carrot extract, horse chestnut extract, peach extract, peach leaf extract, mulberry extract, knapweed extract, hamamelis extract, placenta extract, thymic extract, silk extract, and licorice extract.

Examples of other components include, but are not particularly restricted to, vitamins such as vitamin A oil, retinol, retinol palmitate, inositol, pyridoxine hydrochloride, benzyl nicotinate, nicotinamide, DL-α-tocopherol nicotinate, magnesium ascorbyl phosphate, 2-O-α-D-glucopyranosyl-L-ascorbic acid, vitamin D2 (ergocalciferol), DL-α-tocopherol, DL-α-tocopherol acetate, pantothenic acid, and biotin; hormones such as estradiol and ethynyl estradiol; amino acids such as arginine, aspartic acid, cystine, cysteine, methionine, serine, leucine, and tryptophan; anti-inflammatory agents such as allantoin and azulene; whitening agents such as arbutin; astringents such as zinc oxide and tannic acid; inorganic pigments such as titanium dioxide, red oxide, iron titanate, γ-iron oxide, yellow ocher, yellow iron oxide, black iron oxide, and carbon black; fatty acids such as lauric acid, myristic acid, palmitic acid, stearic acid, oleic acid, and isostearic acid, as well as sodium salts, potassium salts, calcium salts, magnesium salts, strontium salts, barium salts, and zinc salts thereof; refrigerants such as L-menthol and camphor, sulfur, lysozyme chloride, and pyridoxine chloride; and silicone oil.

### EXAMPLES

Hereinafter, the present invention will be explained with reference to examples. However, the present invention is not limited to these examples.

### Method for Measuring Average Particle Diameter

In the examples and comparative examples, the average particle diameter of zinc oxide was measured using a laser diffraction/scattering particle size distribution analyzer. The analyzer used was Model LS 13 320 manufactured by Beckman Coulter, Inc. The median size (D50) was used as the average particle diameter.

### Production of Zinc Oxide Particles

### (Example 1)

Zinc Oxide No. 1 (manufactured by Sakai Chemical Industry Co., Ltd.) was placed in a rectangular sagger and calcinated in an electric furnace at 1000°C to give large-sized zinc oxide particles. The obtained zinc oxide particles had an average particle diameter of 10.7 µm.

### (Example 2)

Zinc Oxide No. 1 (manufactured by Sakai Chemical Industry Co., Ltd.) was placed in a rectangular sagger and calcinated in an electric furnace at 850°C to give large-sized zinc oxide particles. The obtained zinc oxide particles had an average particle diameter of 5.2 µm.

The zinc oxide particles of Examples 1 and 2, LPZINC-2 (manufactured by Sakai Chemical Industry Co., Ltd., average particle diameter: 1.9 µm, Comparative Example 1), Zinc Oxide No. 1 (manufactured by Sakai Chemical Industry Co., Ltd., average particle diameter: 0.75 µm, Comparative Example 2), and fine zinc oxide (manufactured by Sakai Chemical Industry Co., Ltd., average particle diameter: 0.12 µm, Comparative Example 3) were measured for average friction coefficient. The results are shown in Table 1. Incidentally, the measurement method is as follows.

### Method for Measuring Average Friction Coefficient

A 25-mm-wide double-stick tape was stuck on a slide glass, and a powder was placed thereon and spread by a makeup puff. The average friction coefficient under a load of 25 g was measured using a friction tester (Model KES-SE) manufactured by Kato Tech Co., Ltd.

**Table 1**

| Sample | Average Friction Coefficient |
|---|---|
| Zinc oxide particles of Example 1 | 1.82 |
| Zinc oxide particles of Example 2 | 2.35 |
| Zinc oxide particles of Comparative Example 1 | 3.11 |
| Zinc oxide particles of Comparative Example 2 | 6.24 |
| Zinc oxide particles of Comparative Example 3 | 6.70 |

The zinc oxide particles of Examples 1 and 2, the zinc oxide particles of Comparative Examples 1 to 3, and a heat-shielding pigment (manufactured by TAYCA Corporation, Titanium Oxide MP-100, Comparative Example 4) were measured for total visible light transmittance. The results are shown in Table 2. Incidentally, the measurement method is as follows.

### Method for Measuring Visible Light Transmittance

In a 75-ml mayonnaise bottle, 2 g of zinc oxide particles (or titanium oxide particles), 10 g of an acrylic polyol resin, 5 g of xylene, 5 g of butyl acetate, and 38 g of glass beads (φ 1.5 mm) were placed and shaken with a paint conditioner for 90 minutes to give a dispersion. The obtained dispersion was applied to a slide glass using a bar coater #6, and the total light transmittance was measured at wavelengths of 400 nm and 550 nm using a spectrophotometer (manufactured by JASCO Corporation, Model V-570).

**Table 2**

| Sample | Total Light Transmittance at Wavelength of 400 nm (%) | Total Light Transmittance at Wavelength of 550 nm (%) |
|---|---|---|
| Zinc oxide particles of Example 1 | 90 | 95 |
| Zinc oxide particles of Example 2 | 90 | 95 |
| Zinc oxide particles of Comparative Example 1 | 82 | 90 |
| Zinc oxide particles of Comparative Example 2 | 72 | 83 |
| Zinc oxide particles of Comparative Example 3 | 67 | 84 |
| Titanium oxide particles of Comparative Example 4 | 51 | 58 |

The zinc oxide particles of Examples 1 and 2, the zinc oxide particles of Comparative Examples 1 to 3, and the titanium oxide particles of Comparative Example 4 were measured for near-infrared reflectance. The results are shown in Table 3. Incidentally, the measurement method is as follows.

### Method for Measuring Infrared Reflectance

Zinc oxide particles were placed in an aluminum cap 35 mm in diameter and about 10 mm in depth, and pressed by a pressing machine for 20 seconds under a load of 15 tf. The resulting test piece was measured for spectral reflectance at wavelengths of 1000 nm and 2000 nm using a spectrophotometer (manufactured by JASCO Corporation, Model V-570).

**Table 3**

| Sample | Spectral Reflectance (%) | |
|---|---|---|
| | 1000 nm | 2000 nm |
| Zinc oxide particles of Example 1 | 91 | 82 |
| Zinc oxide particles of Example 2 | 94 | 90 |
| Zinc oxide particles of Comparative Example 1 | 97 | 91 |
| Zinc oxide particles of Comparative Example 2 | 99 | 89 |
| Zinc oxide particles of Comparative Example 3 | 95 | 37 |
| Titanium oxide particles of Comparative Example 4 | 89 | 79 |

### (Production of Cosmetics)

According to the formulation shown in Table 4 below, components (1) to (10) were mixed to give a mixture A. Subsequently, components (11) to (13) were mixed to give a mixture B. The obtained mixtures A and B were uniformly mixed and then pressed on a metal plate to give cosmetics (Examples 3 and 4 and Comparative Examples 5 to 8).

### (Evaluation of Cosmetics)

The obtained cosmetics were evaluated by ten panelists according to the following standards.
⊙: Excellent, ○: Good, Δ: Fair, x: Poor

**Table 4**

| | | Example 3 | Example 4 | Comparative Example 5 | Comparative Example 6 | Comparative Example 7 | Comparative Example 8 |
|---|---|---|---|---|---|---|---|
| (1) | Zinc oxide particles of Example 1 | 20 | - | - | - | - | - |
| | Zinc oxide particles of Example 2 | - | 20 | - | - | - | - |
| | Zinc oxide particles of Comparative Example 1 | - | - | 20 | - | - | - |
| | Zinc oxide particles of Comparative Example 2 | - | - | - | 20 | - | - |
| | Zinc oxide particles of Comparative Example 3 | - | - | - | - | 20 | - |
| (2) | Talc | 16.5 | 16.5 | 16.5 | 16.5 | 16.5 | 36.5 |
| (3) | Sericite | 30 | 30 | 30 | 30 | 30 | 30 |
| (4) | Titanium dioxide | 8.5 | 8.5 | 8.5 | 8.5 | 8.5 | 8.5 |
| (5) | Platy barium sulfate | 7 | 7 | 7 | 7 | 7 | 7 |
| (6) | Silicone powder | 5 | 5 | 5 | 5 | 5 | 5 |
| (7) | Zinc myristate | 1.5 | 1.5 | 1.5 | 1.5 | 1.5 | 1.5 |
| (8) | Yellow iron oxide | 1.6 | 1.6 | 1.6 | 1.6 | 1.6 | 1.6 |
| (9) | Red iron oxide | 0.4 | 0.4 | 0.4 | 0.4 | 0.4 | 0.4 |
| (10) | Brown iron oxide | 0.5 | 0.5 | 0.5 | 0.5 | 0.5 | 0.5 |
| (11) | Acrylic-modified silicone | 2 | 2 | 2 | 2 | 2 | 2 |
| (12) | Squalane | 3 | 3 | 3 | 3 | 3 | 3 |
| (13) | Silicone gel | 4 | 4 | 4 | 4 | 4 | 4 |
| Total | | 100 | 100 | 100 | 100 | 100 | 100 |
| Using comfort | | ⊙ | ⊙ | ○ | Δ | × | Δ |
| Transparency | | ⊙ | ○ | Δ | × | Δ | Δ |
| Cooling sensation | | ⊙ | ⊙ | ⊙ | Δ | Δ | ○ |

From Table 4, it was shown that according to the present invention, a cosmetic that is comfortable to use and has excellent transparency and cooling sensation is obtained.

### Industrial Applicability

The present invention has made it possible to provide zinc oxide particles that satisfy all of excellent using comfort, transparency, and infrared reflection properties. Use of the zinc oxide particles of the present invention makes it possible to provide a cosmetic that is comfortable to use, has excellent transparency and cooling sensation, and lasts long.

## Claims

1. Zinc oxide particles having an average particle diameter of 3 to 20 µm, an average friction coefficient of 3 or less, a total visible light transmittance of 85% or more, and a near-infrared reflectance of 80% or more.

2. A cosmetic comprising the zinc oxide particles according to claim 1.
